# FASCICULE DE BREVET EUROPEEN

(11) **EP 0 697 207 B1**
(45) Date de publication et mention de la délivrance du brevet: **09.10.1996**
(21) Numéro de dépôt: 95401310.8
(22) Date de dépôt: 06.06.1995
(51) Int. Cl.: A61K 7/00, A61K 7/48

(54) **Composition gommante contenant des particules de polymère expansé**
Expandierte Polymer-Partikel enthältende Peelingzusammensetzung
Peeling composition containing expanded polymer particles

(30) Priorité: 11.07.1994 FR 9408562
(43) Date de publication de la demande: 21.02.1996
(73) Titulaire: L'OREAL, F-75008 Paris (FR)
(72) Inventeur: Bara, Isabelle, F-75013 Paris (FR); Mellul, Myriam, F-94240 L'Hay-les-Roses (FR)
(74) Mandataire: Dodin, Catherine

(56) Documents cités:
- EP-A- 0 502 769
- EP-A- 0 566 442
- DE-B- 2 521 003

## Description

L'invention se rapporte à une composition gommante utilisable notamment dans les domaines cosmétique et/ou dermatologique pour masser, nettoyer en profondeur et/ou gommer la peau du visage, du cou et/ou du corps humain. En particulier, cette composition permet l'élimination, tout en douceur, des cellules mortes et impuretés de la peau, ainsi que le débouchage des pores de la peau.

L'invention s'applique à tout type de composition et notamment à des émulsions eau-dans-huile ou huile-dans-eau, à des compositions anhydres comme les pâtes ou produits coulés, à des gels lipophiles et à des compositions liquides lipophiles.

L'invention a aussi pour objet un procédé pour masser, nettoyer et/ou gommer la peau du corps, du cou et/ou du visage.

Les produits gommants ou exfoliants, connus aussi sous le nom de "scrub", ont pour but de rendre la peau du visage, du corps et/ou du cou plus douce, mieux préparée pour l'application d'un produit notamment de soin, de maquillage, ou d'auto-bronzant. Ils se présentent généralement sous forme d'émulsions comme dans le document CH-678 488. Ces émulsions contiennent comme particules exfoliantes de la poudre de polyéthylène, de fines particules de quartz, de noyaux ou de graines végétales (coques de noix par exemple). Ces particules ont généralement des diamètres moyens allant de 300 µm à 800 µm.

Malheureusement, du fait de la nature (dure, rugueuse) et/ou de la taille même des particules exfoliantes utilisées, ces produits exfoliants connus présentent généralement l'inconvénient d'être abrasifs, irritants et d'être mal supportés par les peaux sensibles. En outre, ces produits classiques provoquent des dessèchements de la peau notamment par suite d'une élimination trop forte de la barrière hydrolipidique, proctectrice de l'épiderme, ce qui nécessite, après leur application sur la peau, l'utilisation d'une crème nutritive, protectrice et/ou hydratante.

On a envisagé dans la demande de brevet français 93/00990, la réalisation d'un gel aqueux gommant exempt de matières grasses et contenant des microsphères de copolymère de méthacrylate de méthyle, d'acrylonitrile et/ou de chlorure de vinylidène ayant une granulométrie allant de 80 µm à 250 µm. Du fait de l'absence de matières grasses, ce gel, peut dans certains cas, tirailler, voire dessécher la peau, malgré la présence des microsphères.

Il subsiste donc le besoin d'une composition du type "2 en 1" ne nécessitant pas de traitement complémentaire de la peau après gommage ni ne conférant de toucher collant ni de dessèchement de la peau.

L'invention a justement pour objet une composition gommante, caractérisée en ce qu'elle comprend, dans un milieu cosmétiquement et/ou dermatologiquement acceptable contenant des matières grasses, des particules creuses déformables ayant une granulométrie allant de 80 µm à 300 µm et une masse volumique allant de 15 kg/m³ à 200 kg/m³.

Par "particules déformables", il faut comprendre des particules souples et élastiques, qui, après écrasement, reprennent leur forme initiale.

Par "matière grasse", il faut comprendre des composés lipophiles comme les huiles et/ou les composés cireux.

De façon avantageuse, ces particules ont une granulométrie allant de 80 µm à 250 µm, et mieux de 120 µm à 250 µm, voire de 150 µm à 220 µm.

De façon avantageuse, les particules présentent une masse volumique allant de 15 kg/m³ à 200 kg/m³ et mieux allant de 15 kg/m³ à 150 kg/m³. De façon surprenante, la faible masse volumique des particules contribuent, avec leur granulométrie, à la diminution du toucher gras et à l'augmentation de la douceur de la composition de l'invention. Cette caractéristique n'est ni décrite ni suggérée dans l'état de la technique.

Malgré la faible taille des particules, la composition de l'invention assure un gommage efficace et ce, sans effet agressif, du fait de leur déformabilité.

Selon l'invention, il est possible de réaliser une composition quasi-anhydre, contenant moins de 10 % d'eau. Avec ce faible pourcentage d'eau, il est tout-à-fait surprenant de n'obtenir aucun toucher gras ni collant sur la peau. Par ailleurs, la composition de l'invention permet de gommer la peau tout en l'hydratant, la nourrissant et/ou la protégeant.

En plus de ces avantages, la composition de l'invention présente une très grande douceur à l'application. Son aspect est, en outre, très attrayant.

Toutes les qualités de la composition de l'invention sont liées à la déformabilité des particules et à leur faible densité, qui, lorsque ces sphères sont utilisées à poids égal aux particules classiques, donnent des produits beaucoup plus attractifs et efficaces.

Pour obtenir cette masse volumique, on utilise avantageusement des particules de polymères ou copolymères expansés de préférence à base de chlorure de vinylidène, d'acrylonitrile, de chlorure de vinyle, d'un monomère acrylique ou styrénique ou à base d'un mélange de ces monomères.

On peut, par exemple, utiliser un copolymère contenant : de 0 % à 60 % de motifs dérivés du chlorure de vinylidène, de 20 % à 90 % de motifs dérivés d'acrylonitrile et de 0 % à 50 % de motifs dérivés d'un monomère acrylique ou styrénique, la somme des pourcentages (en poids) étant égale à 100. Le monomère acrylique est par exemple un acrylate ou méthacrylate de méthyle ou d'éthyle. Le monomère styrénique est par exemple l'α-méthyl-styrène ou le styrène. De préférence le troisième monomère est le méthacrylate de méthyle. Ces particules peuvent se présenter à l'état sec ou hydraté. En particulier, les particules sont réalisées en méthacrylate de méthyle/acrylonitrile ou méthacrylate de méthyle/acrylonitrile/chlorure de vinylidène expansé.

De façon générale, les particules utilisables dans l'invention peuvent être réalisées en tout matériau thermoplastique, expansé, non toxique et non irritant pour la peau. Elle se présentent de préférence sous forme de microsphères.

Les particules de l'invention peuvent être obtenues, par exemple, selon les procédés des brevets et demandes de brevet EP-56 219, EP-348 372, EP-486 080, EP-320 473, EP-112 807 et US-3 615 972.

La cavité interne des particules contient en principe un gaz qui peut être de l'air, de l'azote ou un hydrocarbure comme l'isobutane ou l'isopentane.

Les particules creuses utilisables dans l'invention sont, en particulier, celles vendues sous la marque EXPANCEL par la Société Nobel Casco, avec la qualité DE comme le produit 551 DE 80 de 80 µm environ de granulométrie.

Dans les compositions de l'invention, on utilise de préférence de 0,1 % à 10 % en poids de particules et mieux de 0,3 % à 5 % et encore mieux de 0,3 % à 1,5 %, par rapport au poids total de la composition. Du fait de la faible densité des particules, on peut incorporer un grand volume de particules pour un faible poids.

Un autre avantage des particules ci-dessus, est qu'elles peuvent être utilisées en quantité plus faible que les particules classiquement utilisées (2 % à 5 % pour la poudre de polyéthylène), du fait de leur densité extrêmement basse.

De ce fait, les produits de l'invention pourront être utilisés, quotidiennement, comme nettoyants ou exfoliants doux ou comme produits de massage du visage et/ou du corps.

Grâce aux particules déformables et à leur action mécanique, la composition de l'invention confère un massage plus efficace que les huiles ou corps gras, sans particules, généralement utilisés pour le massage.

La composition de l'invention peut être une émulsion eau-dans-huile ou huile-dans-eau, un gel lipophile, une pâte ou un produit coulé anhydre ou à forte teneur en corps gras. En résumé, la composition de l'invention peut se présenter sous toute forme galénique contenant des matières grasses, ces dernières "fixant" les particules à la surface de la peau et empêchant leur élimination au cours du massage.

Selon l'invention, la composition peut contenir de 5 % à 99,9 % en poids de matières grasses par rapport au poids total de la composition, et mieux, de 10 % à 50 % en poids. La quantité d'huile dépend en fait de la forme galénique.

Les huiles et les composés cireux utilisables dans l'invention peuvent être d'origine minérale, végétale, synthétique, siliconée ou fluorée. Ils sont ceeux classiquement utilisés dans le domaine cosmétique et/ou dermatologique.

A titre d'exemples, on peut citer comme huile minérale, l'huile de paraffine ou de vaseline ; comme huile végétale, l'huile d'amande douce, d'avocat, de ricin, d'olive, de pépin de cassis, de palme, de germe de blé, le beurre de karité ; comme huile synthétique, les triglycérides d'acides caprylique-caprique, I'octyldodécanol, l'alcool cétylique, l'acide stéarique ou stéarylique et les esters gras tels que le myristate de butyle ou le palmitate d'isopropyle ou d'octyle, ou le cétéaryl octanoate et myristate d'isopropyle ; comme huile siliconée, les cyclométhicones ou les polydiméthylsiloxanes.

Comme composés cireux utilisables dans l'invention, on peut citer la paraffine, l'huile de jojoba, la cire de carnauba.

Pour obtenir un gel, il est nécessaire d'utiliser des gélifiants hydrophiles ou lipophiles. Ces gélifiants sont ceux classiquement utilisés dans le domaine cosmétique et/ou dermatologique.

Comme gélifiants, on peut citer les argiles modifiées (bentones) et les sels métalliques d'acides gras (stéarate d'aluminium, laurylsulfate d'ammonium) ; les dérivés de la cellulose, les polymères polycarboxyvinyliques, les alcools gras.

Dans le cas d'une émulsion, on peut utiliser des tensioactifs pour disperser les matières grasses dans de l'eau pour réaliser des émulsions huile/eau ou inversement, pour disperser l'eau dans les matières grasses pour réaliser des émulsions eau/huile.

Comme tensioactifs, on peut utiliser les esters d'acide gras et de glycérol ou de polyéthylène glycol (stéarate de glycéryle ou de polyéthylène glycol) ou de sucre (stéarate de sorbitane).

Les compositions huileuses de l'invention peuvent, en outre, contenir divers ingrédients classiquement utilisés dans les domaines cosmétique et/ou dermatologique. Ces ingrédients peuvent consister en des actifs hydrophiles, des actifs lipophiles, des parfums, des conservateurs, des matières colorantes (pigments ou colorants), des solvants (alcools inférieurs), des argiles ou encore des agents de texture comme des agents pulvérulents autres que les particules exfoliantes. Ces ingrédients additionnels sont utilisés en quantité habituelle.

Comme actifs hydrophiles, on peut citer les polyols tels que le propylène glycol, la glycérine, le sorbitol. Comme actifs lipophiles, on peut citer les huiles essentielles ou les huiles démaquillantes comme le palmitate d'éthyl-2 hexyle, les filtres absorbant les ultraviolets, les vitamines.

L'invention a aussi pour objet un procédé pour nettoyer et/ou gommer la peau du corps et/ou du visage, y compris le cou, consistant à appliquer sur la peau une composition telle que définie précédemment, à masser la peau avec cette composition pour éliminer les cellules mortes puis à rincer la peau, généralement à l'eau, en vue d'éliminer ces cellules mortes et les particules.

L'invention a aussi pour objet l'utilisation de la composition définie précédemment pour nettoyer et/ou gommer la peau du visage, du cou et/ou du corps humain.

L'invention a encore pour objet une utilisation de particules creuses déformables dans un milieu cosmétiquement et/dermatologiquement acceptable contenant des matières grasses, pour nettoyer et/ou gommer la peau du visage, du cou et/ou du corps humain, ces particules creuses déformables ayant une granulométrie comprise entre 80 µm et 300 µm et une masse volumique allant de 15 kg/m³ à 200 kg/m³.

Le nettoyage du corps humain concerne aussi bien les jambes, que les bras, le dos et le ventre.

Compte-tenu des propriétés non irritantes de la composition de l'invention, celle-ci peut être utilisée quotidiennement même par les personnes à peau très sèche.

Les exemples qui suivent sont donnés à titre illustratif en vue de mieux faire comprendre l'invention. Les quantités sont données en % en poids.

### Exemple 1 : Scrub

| *Phase A : grasse* | |
|---|---|
| - Stéarate de glycérol et PEG * | |
| 100 (tensioactif) | 1,2 % |
| - Polyéthylène glycol 20 stéarate (tensioactif) | 1,2 % |
| - Acide stéarique | 0,6 % |
| - Alcool stéarylique | 0,6 % |
| - Alcool cétylique | 0,6 % |
| - Cétéaryl octanoate et myristate d'isopropyle | 3 % |
| - Huile de vaseline | 15,1 % |

| *Phase B : aqueuse* | |
|---|---|
| - Glycérol | 3 % |
| - Conservateur | 0,5 % |
| - Eau distillée | qsp 100 % |

| *Phase C :* | |
|---|---|
| - Microsphères creuses de terpolymère ** | 2 % |

| | |
|---|---|
| (*) PEG signifie polyéthylèneglycol | |
| (**) Particules d'acrylonitrile/méthacrylate de méthyle/chlorure de vinylidène expansé, de granulométrie moyenne de 210 µm± 5 µm et de masse volumique de 25 kg/m³ ± 2. | |

### Mode opératoire :

Chauffage de la phase A à 80 °C. Chauffage de la phase B à 80 °C. Emulsion en versant B dans A sous agitation forte à l'aide d'une turbine à 80 °C. Incorporation de C dans l'émulsion sous agitation très lente, à 80 °C.

On obtient un produit très attractif contenant des petites sphères bien visibles, procurant un effet "Scrub", très doux au toucher.

### Exemple 2 : Produit de nettoyage pour le visage

| | |
|---|---|
| - Conservateur | 0,3 % |
| - Acide stéarique | 4 % |
| - Stéarate de glycérol (tensioactif) | 3 % |
| - Microsphères creuses de terpolymère * | 1 % |
| - Alcool cétylique | 4 % |
| - Lauryl sulfate d'ammonium (tensioactif) | 5,83 % |
| - Triglycérides d'acides caprylique-caprique | 5,5 % |
| - Propylène glycol (hydratant) | 4,5 % |
| - Eau distillée | qsp 100 % |

| | |
|---|---|
| (*) Particules d'acrylonitrile/méthacrylate de méthyle/chlorure de vinylidène expansé de granulométrie moyenne de 185 µm ± 5 et de masse volumique de 44 kg/m³ ± 2 | |

### Mode opératoire :

On mélange tout d'abord les corps gras et les tensioactifs à chaud. Puis on verse cette phase grasse dans la phase aqueuse. Les particules sont introduites en dernier.

On obtient un produit d'aspect attractif, de nettoyage pour le visage et non agressif. Le nettoyage grâce à la présence des microsphères de terpolymère est approfondi. La peau traitée est lisse, douce et bien hydratée.

## Revendications

1. Utilisation d'une composition huileuse comprenant, dans un milieu cosmétiquement et/ou dermatologiquement acceptable contenant des matières grasses, des particules creuses déformables ayant une granulométrie comprise entre 80 µm et 300 µm et une masse volumique allant de 15 kg/m³ à 200 kg/m³ pour gommer la peau du visage, du cou et/ou du corps humain.

2. Utilisation selon la revendication 1, caractérisée en ce que les particules ont une granulométrie comprise allant de 120 µm et 250 µm.

3. Utilisation selon l'une des revendications précédentes, caractérisée en ce que les particules sont réalisées en un matériau thermoplastique expansé.

4. Utilisation selon l'une des revendications précédentes, caractérisée en ce que les particules sont en polymère ou copolymère de méthacrylate de méthyle et/ou d'acrylonitrile et/ou de chlorure de vinylidène.

5. Utilisation selon l'une des revendications précédentes, caractérisée en ce que les particules sont en copolymère d'acrylonitrile et de méthacrylate de méthyle et/ou de chlorure de vinylidène.

6. Utilisation selon l'une des revendications précédentes, caractérisée en ce que les particules représentent de 0,3 % à 5 % du poids total de la composition.

7. Utilisation selon l'une des revendications précédentes, caractérisée en ce que les matières grasses représentent de 5 % à 99,9 % du poids total de la composition.

8. Utilisation selon l'une des revendications précédentes, caractérisée en ce que la composition contient, en outre, au moins un adjuvant choisi parmi les gélifiants, les agents tensioactifs, les actifs hydrophiles, les actifs lipophiles, les parfums, les conservateurs, les solvants, les matières colorantes, les cires, les argiles, les agents de texture.

9. Utilisation selon l'une des revendications précédentes, caractérisée en ce que la composition se présente sous forme d'une émulsion eau-dans-huile ou huile-dans-eau, d'un gel lipophile, d'une pâte ou d'un produit coulé anhydre.

## Claims

1. Use of an oily composition including, in a cosmetically and/or dermatologically acceptable medium containing fatty matter, deformable hollow particles which have a particle size of between 80 µm and 300 µm, and a density ranging from 15 kg/m³ to 200 kg/m³, for deep-cleansing the skin of the face, of the neck and/or of the human body.

2. Use according to Claim 1, characterized in that the particles have a particle size ranging from 120 µm to 250 µm.

3. Use according to one of the preceding claims, characterized in that the particles are made of an expanded thermoplastic material.

4. Use according to one of the preceding claims, characterized in that the particles are made of polymer or copolymer of methyl methacrylate and/or of acrylonitrile and/or of vinylidene chloride.

5. Use according to one of the preceding claims, characterized in that the particles are made of copolymer of acrylonitrile and of methyl methacrylate and/or of vinylidene chloride.

6. Use according to one of the preceding claims, characterized in that the particles represent from 0.3 % to 5 % of the total weight of the composition.

7. Use according to one of the preceding claims, characterized in that the fatty matter represents from 5 % to 99.9 % of the total weight of the composition.

8. Use according to one of the preceding claims, characterized in that the composition additionally contains at least one adjuvant chosen from gelling agents, surface-active agents, hydrophilic active substances, lipophilic active substances, perfumes, stabilizers, solvents, colorant matter, waxes, clays and texture agents.

9. Use according to one of the preceding claims, characterized in that the composition is in the form of a water-in-oil or oil-in-water emulsion, of a lipophilic gel, of a paste or of an anhydrous cast product.

## Patentansprüche

1. Verwendung einer öligen Zusammensetzung, die in einem kosmetisch und/oder dermatologisch akzeptablen Medium Fettsubstanzen und verformbare Hohlpartikel mit einer Teilchengröße im Bereich von 80 bis 300 µm und einer Dichte im Bereich von 15 bis 200 kg/m³ enthält, zur abradierend wirkenden Behandlung der Haut des Gesichts, des Halses und/oder des menschlichen Körpers.

2. Verwendung nach Anspruch 1, dadurch gekennzeichnet, daß die Partikel eine Teilchengröße im Bereich von 120 bis 250 µm aufweisen.

3. Verwendung nach einem der vorhergehenden Ansprüche, dadurch gekennzeichnet, daß die Partikel aus einem expandierten thermoplastischen Material hergestellt werden.

4. Verwendung nach einem der vorhergehenden Ansprüche, dadurch gekennzeichnet, daß die Partikel aus einem Polymer oder Copolymer aus Methylmethacrylat und/oder Acrylnitril und/oder Vinylidenchlorid bestehen.

5. Verwendung nach einem der vorhergehenden Ansprüche, dadurch gekennzeichnet, daß die Partikel aus einem Copolymer aus Acrylnitril und Methylmethacrylat und/oder Vinylidenchlorid bestehen.

6. Verwendung nach einem der vorhergehenden Ansprüche, dadurch gekennzeichnet, daß der Mengenanteil der Partikel 0,3 bis 5 Gew.-% des Gesamtgewichts der Zusammensetzung beträgt.

7. Verwendung nach einem der vorhergehenden Ansprüche, dadurch gekennzeichnet, daß der Mengenanteil der Fettsubstanzen 5 bis 99,9 % des Gesamtgewichts der Zusammensetzung beträgt.

8. Verwendung nach einem der vorhergehenden Ansprüche, dadurch gekennzeichnet, daß die Zusammensetzung ferner mindestens einen Hilfsstoff enthält, der unter Gelbildnern, grenzflächenaktiven Stoffen, hydrophilen Wirkstoffen, lipophilen Wirkstoffen, Parfums, Konservierungsmitteln, Färbemitteln, Wachsen, Tonen und texturbeeinflussenden Mitteln ausgewählt wird.

9. Verwendung nach einem der vorhergehenden Ansprüche, dadurch gekennzeichnet, daß die Zusammensetzung in Form einer Wasser-in-Öl-Emulsion, einer Öl-in-Wasser-Emulsion, eines lipophilen Gels, einer Paste oder eines wasserfreien gegossenen Produkts vorliegt.
